**Europäisches Patentamt**

(19) **European Patent Office**

**Office européen des brevets**

(11) Veröffentlichungsnummer: **0 080 193**
**B1**

(12) # EUROPÄISCHE PATENTSCHRIFT

(45) Veröffentlichungstag der Patentschrift:
14.01.87

(51) Int. Cl.⁴: **A 61 F 5/04**

(21) Anmeldenummer: **82110739.8**

(22) Anmeldetag: **20.11.82**

(54) Fixationsvorrichtung für ein Extensions- und Repositionsgerät zur Behandlung von Knochenbrüchen.

(30) Priorität: **20.11.81 DE 8133813 U**

(43) Veröffentlichungstag der Anmeldung:
**01.06.83 Patentblatt 83/22**

(45) Bekanntmachung des Hinweises auf die Patenterteilung:
**14.01.87 Patentblatt 87/3**

(84) Benannte Vertragsstaaten:
**AT IT**

(56) Entgegenhaltungen:
**DE-A-2 602 228**
**DE-A-2 843 711**
**DE-C-742 951**
**US-A-3 010 452**
**US-A-3 027 895**
**US-A-4 123 806**

**PENSEZ PLASTIQUES, INDUSTRIES, Band 27, 1963, Seite 316.,23. "Nouveaux gants résistants"**

(73) Patentinhaber: **Ruf, Hermann, Pfützenstrasse 58, D-6103 Griesheim (DE)**

(72) Erfinder: **Ruf, Hermann, Pfützenstrasse 58, D-6103 Griesheim (DE)**

(74) Vertreter: **Zinngrebe, Horst, Dr.rer.nat., Saalbaustrasse 11, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Fixationsvorrichtung zur Verwendung an einem Extensions- und Repositionsgerät zum Einrichten einer Extremität, bestehend aus einer Stützgabel aus einem sterilisierbaren, röntgendurchlässigen Kunststoff für die Extremität und einem Verbindungselement, welches an seinen beiden freien Enden je ein Kupplungsteil aufweist, und wobei die Stützgabel aus einem unteren Stiel, an dem Halteteile zum Festlegen der Stützgabel an einem Support des Gerätes befestigt sind, sowie einem seitlich vorstehenden Stützarm und einem sich im wesentlichen parallel zum Stiel erhebenden Anlagearm besteht, wobei an dem freien Ende des Anlagearmes und an dem freien Ende des Stützarmes je ein Gegenkupplungsteil ausgebildet sind.

In der deutschen Offenlegungsschrift 25 02 228 ist ein Extensionsgerät und Repositionsgerät zum Einrichten und Behandeln von Knochenbrüchen, insbesondere für die geschlossene Marknagelung mit Anwendung moderner Röntgenanlagen beschrieben, an dessen Support eine aus sterilisierbaren, röntgendurchlässigen Kunststoff bestehende Stützgabel befestigt ist. Das grundsätzliche Prinzip dieses Gerätes lässt sich auf den von Dr. R. Wittmoser im Jahre 1943 entwickelten Extensionstisch zurückführen.

Bei den früheren Ausführungsformen des Extensionstisches wurden statt der Stützgabel Halteringe aus Holz verwendet, die jedoch nicht sterilisierbar sind und aus einer großen Anzahl handgefertigter Einzelteile bestehen. Die sterilisierbare Stützgabel gemäß der erwähnten Offenlegungsschrift kann durch Anlegen verstellbarer Kunststoffriemen, geschlossen werden, die um die Extremität gelegt werden und diese am Stützarm und Anlagearm festlegen. Die Verwendung der Kunststoffriemen hat den Nachteil, daß diese sich nur schwer sterilisieren lassen. Außerdem schneiden sie leicht in die Weichteile der Extremität ein und sind daher für den Patienten unbequem. Ferner lassen sich die Kunststoffriemen nur schwer an einen veränderten Umfang eines anderen Abschnittes der auf der Stützgabel liegenden Extremität nachstellen.

Der Erfindung liegt daher die Aufgabe zugrunde, die eingangs erwähnte Fixationsvorrichtung voll sterilisierbar und so zu gestalten, daß sie in Weichteile nicht einschneidet, und sich leicht an unterschiedliche Dicken der Exremitätpartien anpassen läßt.

Die Fixationsvorrichtung zeichnet sich dazu erfindungsgemäß dadurch aus, daß das Verbindungselement ein starrer, aus einem sterilisierbaren, röntgendurchlässigen Kunststoff bestehender Halbring ist, der den vom Stützarm und dem Anlagearm gebildeten Ringabschnitt zu einem geschlossenen Ring ergänzt und daß die an den freien Enden des Halbringes ausgebildeten Kupplungsteile und die Gegenkupplungsteile als lösbare Steckverbindungen ausgebildet sind. Dadurch kann jede Extremitätpartie ohne Beschwerden für den Patienten definiert an der Stützgabel festgelegt werden. Die Steckverbindung ermöglicht einen leichten Austausch eines Halbringes gegen einen stärker oder geringer konvex gebogenen, dem Umfang der zu fixierenden Extremitätpartie genau angepaßten Halbring, wobei die Supporteinstellung nicht verändert zu werden braucht.

Wenn der Halbring derart nach außen konvex gebogen ist, daß die Richtungen seiner freien Enden einen spitzen Winkel einschließen, kann mit der Fixationsvorrichtung eine sehr starke Extremitätpartie reponiert werden. Für schlanke Extremitätpartien empfiehlt sich ein derart flachgebogener Halbring, daß die Richtungen seiner freien Enden einen stumpfen Winkel einschließen.

Die Zugänglichkeit für die Aufnahmekamera und das Röntgengerät zu der fixierten Extremitätpartie wird in Weiterbildung der Erfindung dadurch verbessert, daß der Anlagearm relativ zum Stiel seitlich in Richtung des Stützarmes versetzt an den Stiel angeformt ist, da der Support kann außerhalb des Aufnahmebereichs der Röntgeneinrichtung sich befindet.

Als Kunststoff empfielt sich reines Polyäthylen natur, das ausgezeichnete Röntgendurchlässigkeit und Sterilisierbarkeit besitzt.

Um eine möglichst breit Auflagefläche für die Extremität zu schaffen, ist der Querschnitt des Stützarmes zweckmäßig ein liegendes Rechteck mit stark abgerundeten Ecken. Die starke Abrundung vermeidet störendes Eindrücken des Stützarms in die Weichteile.

Die Wiedererkennung der Fixationsstelle auf der Röntgenaufnahme wird durch Referenzmarken erleichtert, die in Weiterbildung der Erfindung durch in den Stützarm und gegebenenfalls auch in den Anlagearm eingelassene Metallstücke geschaffen sind. Dabei kann der Stützarm vom Anlagearm auf der Röntgenaufnahme an der unterschiedlichen Anzahl von Referenzmarken erkannt werden, wenn in Weiterbildung der Erfindung in den Stützarm und den Anlagearm eine verschiedene Anzahl von Metallstücken die zweckmäßig aus Kupferstreifen bestehen können, eingelassen sind. Die Metallstücke können auch im Stützarm eine andere Größe haben, beispielsweise breiter oder länger sein, als die Metallstücke im Anlagearm.

Die Austauschbarkeit der Halbringe an der Stützgabel wird in zweckmäßiger Weise dadurch vereinfacht, daß die Kupplungsteile an den Halbring angeformte Ösen mit Augen sind, deren Innendurchmesser im wesentlichen gleich dem Außendurchmesser je eines Zapfens ist, der aus jedem Gegenkupplungsteil vorsteht. Auch kann jedes Gegenkupplungsteil aus einer an den Stützarm und an den Anlagearm angeformten Öse mit Auge bestehen, wobei ein separater

2

Zapfen durch das Auge der Kupplungsteile und der Gegenkupplungsteile steckbar ist. Die Ösen der Kupplungsteile können dann zwischen je ein Ösenpaar am Gegenkupplungsteil bis zur fluchtenden Ausrichtung der Augen eingeführt werden.

Das Einklemmen von Weichteilen im Bereich der Kupplungsteile und Gegenkupplungsteile wird zweckmäßig dadurch mit Sicherheit ausgeschaltet, daß der Stützarm, der Anlagearm und der Halbring neben den Ösen radial einwärts angephast sind, also keine gegeneinander liegende Kanten aufweisen.

Die Erfindung wird nachstehend anhand des in der beigefügten Zeichnung dargestellten Ausführungsbeispiels im einzelnen erläutert. Es zeigen:

Figur 1 eine schematische perspektivische Ansicht eines Extensions- und Repositionsgerätes mit angebauter Fixationsvorrichtung;

Figur 2 eine schematische Seitenansicht einer Stützgabel der Fixationsvorrichtung;

Figur 3 eine schematische Ansicht eines Schnittes durch die Stützgabel nach Figur 2 längs der Linie III-III;

Figur 4 eine schematische Ansicht eines Schnittes durch die Stützgabel nach Figur 2 längs der Linie IV-IV;

Figur 5 eine schematische Seitenansicht eines Halbringes für die Stützgabel nach Figur 2;

Figur 6 eine schematische Stirnansicht des Halbringes nach Figur 5;

Figur 7 eine schematische Seitenansicht der Stützgabel nach Figur 2 mit angebautem Halbring nach Figur 5 und

Figur 8 eine schematische Seitenansicht einer anderen Ausführungsform eines Halbringes.

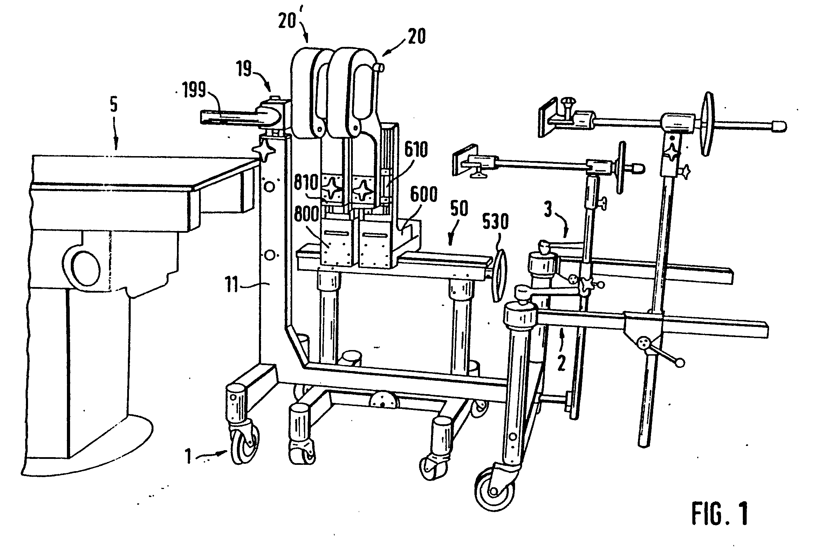

Das fahrbare, gemäß Figur 1 vor der Stirnseite eines Operationstisches 5 aufgestellte Extensions- und Repositionsgerät besteht aus einem im ganzen mit 1 bezeichneten Extensionstisch und einer Repositionslafette 50. Extensionstisch 1 und Repositionslafette 50 ermöglichen zusammen mit der erfindungsgemäßen Fixationsvorrichtung 20 ein definiertes Positionieren und Einrichten etwa eines Beines eines auf dem Operationstisch liegenden Patienten. Der Extensionstisch weist dazu eine Säule 11 auf, an deren oberem Ende 19 ein Auflagebügel 199 etwa für den Oberschenkel vorgesehen ist. Am gegenüberliegenden Ende des Extensionstisches 1 sind an dessen Rahmen zwei seitlich versetzte und jeweils um 360° drehbare Extensionsglieder 2, 3 befestigt, von denen jedes eine Gegenplatte zum Festlegen eines Fußes des Patienten trägt.

Zwischen die Säule 11 und die Extensionsglieder 2, 3 ist die Repositionslafette 50 eingefahren, auf deren Tisch zwei Supporte 600, 800 in Längsrichtung des Operationstisches 5 mittels einer Handkurbel 530 separat oder gemeinsam verschiebbar sind. Jeder Support weist einen Horizontalschlitten und einen Vertikalschlitten auf, wobei an dem

Vertikalschlitten 610 die Fixationsschlitten 810 des Supportes 800 ist eine weitere Fixationsvorrichtung 20' befestigt. Die Fixationsvorrichtungen 20 und 20' sind gleichartig konstruiert, sodaß für die vorliegende Erläuterung nur die Fixationsvorrichtung 20 im einzelnen nachfolgend beschrieben wird. Jede der Fixationsvorrichtungen 20, 20' kann demnach mittels des Handrades 530 sowie mittels des Horizontalschlittens und des Vertikalschlittens individuell in die drei senkrechten Raumrichtungen verfahren werden.

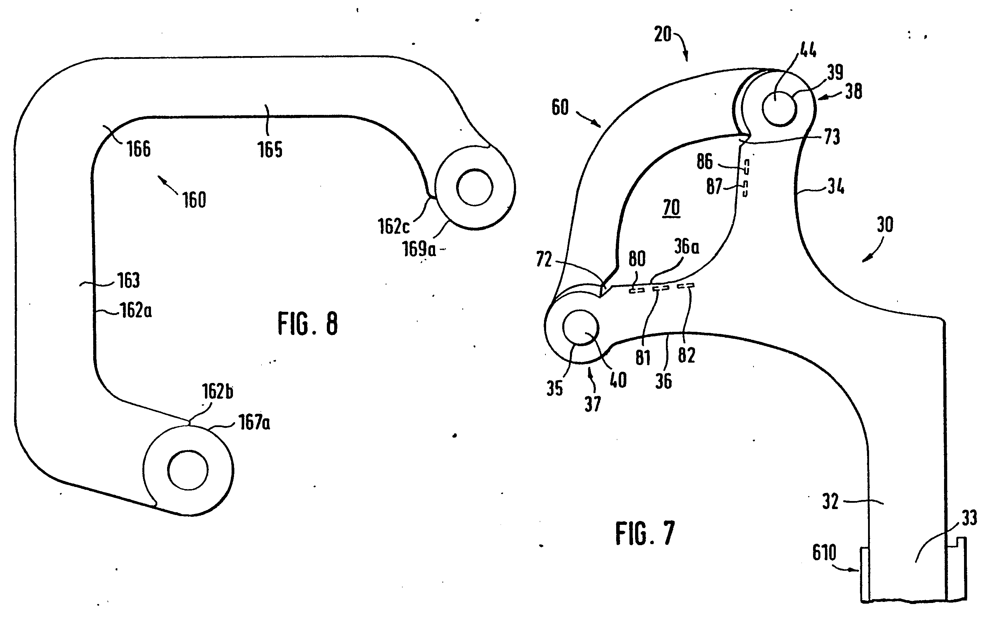

Die Fixationsvorrichtung 20 (Fig. 7) besteht im wesentlichen aus einer Stützgabel 30 und einem Halbring 50. Die Stützgabel 30 weist einen unteren Stiel 32 auf, der an seinem unteren Abschnitt 33 mit dem Vertikalschlitten 610 verbunden ist. Das obere Ende des Stieles 32 verzweigt sich in einen vertikalen, gegenüber dem Stiel 32 nach außen versetzten und sich im wesentlichen parallel zum Stiel erstreckenden Anlagearm 34 sowie in einen horizontal nach außen vorstehenden Stützarm 36. Das obere Ende des Anlagearmes 34 trägt einen ösenartig ausgeformten Gelenkkopf 38 mit einem mittleren Auge 39. Das äußere freie Ende des Stützarmes 36 ist zu einem ähnlichen Gelenkkopf 37 ausgeformt, der ein Auge 35 umschließt.

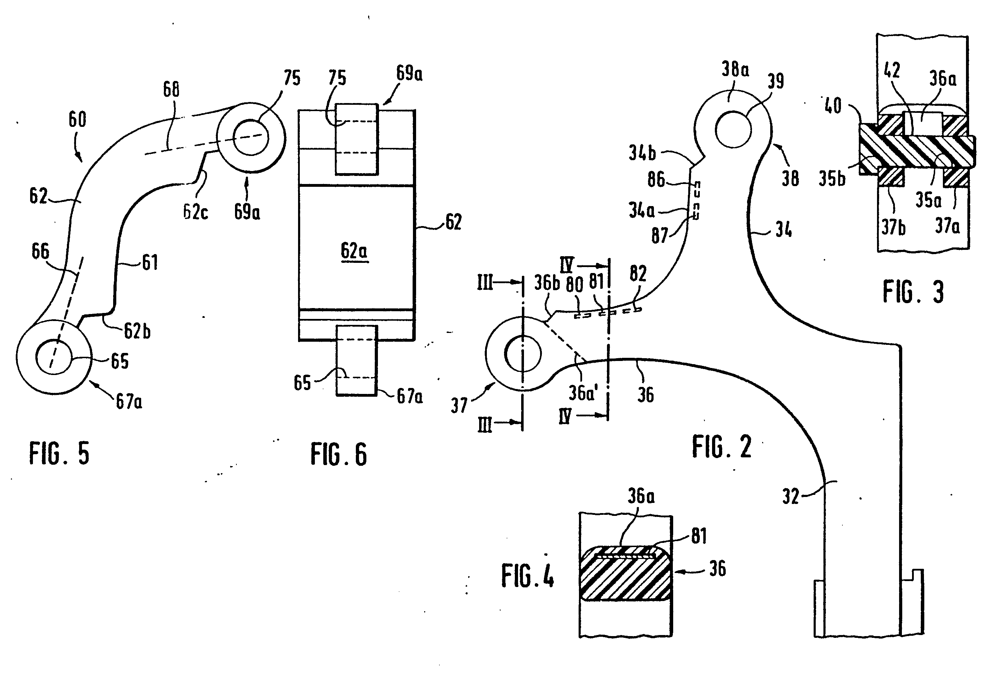

Wie man anhand der Figur 3 erkennt, besteht der Gelenkkopf 37 aus zwei parallelen, beabstandeten Scheiben 37a und 37b, deren fluchtende Durchbohrungen je ein Auge 35a und 35b bilden. Die vordere Stirnfläche 36a', des Stützarmes 36 verläuft nach unten rückwärts (in Fig. 2 gestrichelt angedeutet), um einem zwischen die beiden Scheiben 37a und 37b eingeschobenen scheibenförmigen Kupplungselement 67a eines Halbringes 60 freien Raum zu lassen. Ein Stopfen 40 kann mit seinem Schaft 42 in die Augen 35a und 35b des ein Gegenkupplungsteil bildenden Gelenkkopfes 37 sowie durch das Auge 65 des Kupplungselementes 67a durchgesteckt werden, sodaß auf diese weise der Halbring 60 an einem Ende mit dem Gelenkkopf 37 des Stützarmes 36 drehbar verbunden ist. Durch Herausziehen des Stopfenschaftes 42 aus den Augen 35a, 65 und 35b kann diese Verbindung leicht gelöst werden.

Der Gelenkkopf 38 am oberen Ende des Anlagearmes 34 ist im wesentlichen in gleicher Weise ausgebildet wie der Gelenkkopf 37 des Stützarmes 36. Ein weiterer Stopfen 44 (Figur 7) kann mit seinem Schaft durch das Auge 39 hindurchgeführt werden und ermöglicht im Zusammenwirken mit einem Auge 75 an dem dem Auge 65 gegenüberliegenden freien Ende des Halbringes 60 eine lösbare Steckverbindung des Halbringes 60 mit dem oberen Ende des Anlagearmes 34. Bei der in Figur 2 gewählten Darstellung ist nur die Kupplungsscheibe 38a mit dem Auge 39 zu erkennen, eine parallele weitere Kupplungsscheibe ist mit Abstand hinter der Kupplungsscheibe 38a ausgebildet.

Der Hauptteil 62 des Halbringes 60 (Figuren 5 und 6) hat nur eine schwach konvex gebogene Form, und weist an seinen gegenüberliegenden freien Enden je ein Kupplungselement 67a und 69a auf. Das Kupplungselement 67a und das Kupplungselement 69a ist eine in der Mitte der Breitenerstreckung des Hauptteiles 62 angeformte Scheibe, die mit einem mittigen Auge 65 bzw. 75 versehen ist. Die Stärke der Kupplungselemente 67a und 69a ist geringfügig kleiner als der Abstand der Scheiben 37a und 37b bzw. der das Gegenkupplungsteil bildenden Scheiben des Gelenkkopfes 38, sodaß nach dem Einschieben der Kupplungselemente 67a und 69a zwischen die entsprechenden Scheiben der Gegenkupplungsteile 37 und 38 die jeweiligen Augen fluchten. Wie man insbesondare aus Figur 5 erkennt, ist der Halbring 60 derart schwach konvex, daß die Richtungen 66 und 68 seiner freien Enden einen stumpfen Winkel einschließen. Die Innenkontur 61 des Hauptseiles 62 beschreibt jedoch angenähert einen Viertelkreisbogen.

Wird der Halbring 60 in der in Figur 7 dargestellten Weise mit der Stützgabel 30 verbunden, wobei die Stopfen 40 und 44 in die entsprechenden Augen eingeführt sind, umschließen der Stützarm 36, der Anlagearm 34 und die Innenkontur 61 des Halbringes 60 eine Öffnung 70 und legen eine in der Öffnung 70 befindliche Extremitätenpartie gegen seitliche Verlagerungen fest. Die empfindlichen Weichteile finden dabei eine breite Auflage auf der relativ breiten Auflagefläche 36a des Stützarmes, die an den Seiten gemäß Figur 4 stark abgerundet ist. Dadurch kann die Auflagefläche 36a nicht in die Weichteile einschneiden. Da auch die Anlagefläche 34a des Anlagearmes 34 und die Innenfläche 62a des Hauptteiles 62 genauso breit und seitlich abgerundet sind, können auch durch diese Teile an dem eingespannten Bein oder Arm keine Druckstellen entstehen lassen.

Ferner ist hervorzuheben, daß die Auflagefläche 36a sowie die Innenfläche 62a und die Anlagefläche 34a am Übergang zu den Kupplungselementen 67a und 69a sowie zu den Gelenkköpfen 37 und 38 durch je einen schrägen Anschnitt 36b, 62b, 62c und 34b soweit abgeflacht sind, daß bei auf die Stützgabel 30 aufgesetztem Halbring 60 breite Spalte 72, 73 verbleiben. Diese verhindern, daß beim Schließen der Öffnung 70 durch Aufsetzen des Halbringes 60 auf die Stützgabel 30 Weichteile eingequetscht werden können. Das Einspannen des Beins oder Armes in der Öffnung 70 der geschlossenen, erfindungsgemäßen Fixationsvorrichtung geschieht daher ohne Beeinträchtigung des Befindens des Patienten und ohne nachteilige Folgen für die empfindlichen Weichteile der eingespannten Extremitätenpartie.

Die Stützgabel 30, der Halbring 60 sowie die beiden Stopfen 40 und 44 bestehen aus reinem Polyethylen natur, das eine ausgezeichnete Sterilisierbarkeit und gleichzeitig beste Röntgenstrahldurchlässigkeit garantiert. Um in der fertigen Röntgenaufnahme die auf dem Bild sonst nicht erscheinende Fixationsvorrichtung lokalisieren zu können, sind in den Stützarm 36 und in den Anlagearm 34 dicht unterhalb der Auflagefläche 36a und der Anlagefläche 34a mehrere Kupferstreifen 80, 81, 82 bzw. 86, 87 eingearbeitet, die auf dem Röntgenbild sichtbar werden. Im dargestellten Ausführungsbeispiel sind in den Stützarm 36 drei Kupferstreifen 80, 81 und 82 nebeneinander eingegossen, und in dem Anlagearm 34 sind zur Unterscheidung nur zwei Kupferstreifen 86, 87 übereinander eingebettet. Die Kupferstreifen werden zweckmäßig in entsprechende Öffnungen des Stützarmes 36 und des Anlagearmes 34 seitlich eingedrückt und die Öffnungen anschließend dicht verschlossen, sodaß keine die Sterilisierbarkeit beeinträchtigende Fugen verbleiben.

Ein besonderer Vorteil der Erfindung besteht darin, daß die Stützgabel 30 mit mehreren verschiedenen Halbringen unterschiedlicher Konvexität gekoppelt werden kann, und zwar ohne daß die Stellung des in der Stützgabel liegenden Beines oder Armes oder die Einstellung der Stützgabel 30 relativ zur Repositionslafette 50 geändert werden müssen. So gehört der Halbring 60 zu einer Gruppe von beispielsweise fünf verschiedenen Halbringen unterschiedlicher Krümmung, von denen ein Halbring 160 mit starker Konvexitat in Figur 8 dargestellt ist. Der Halbring 160 trägt an seinen beiden freien Enden scheibenförmige Kupplungselemente 167a und 169a, die in ihrer Form und Anordnung relativ zum Hauptteil 162 des Halbringes 160 den Kupplungselementen 67a und 69a genau entsprechen. Der Hauptteil 162 wird aus zwei im wesentlichen geraden Abschnitten 163 und 165 gebildet, die im wesentlichen rechtwinkelig zueinander verlaufen, durch einen weiten Bogen 166 mit einander verbunden sind und an ihren Enden unter einem Winkel in das entsprechende Kupplungselement 167a bzw. 169a übergehen. Die Innenkontur 162a erhält dadurch eine nach außen gebogene Gestalt, sodaß die Öffnung, die von dem auf die Stützgabel 30 aufgesetzten Halbring 160 mit dem Stützarm 36 und Anlagearm 34 umschlossen wird, im Verhältnis zur Öffnung 70 wesentlich größer ist. Dadurch kann beispielsweise ein Oberschenkel bequem in dieser größeren Öffnung Platz finden. Die Innenfläche des Halbringes 160 ist am Übergang zu den Kupplungselementen 167a und 169a wieder durch Anschnitte 162b, 162c abgeflacht, um ein Einklemmen etwa von Körperhaaren beim Aufsetzen des Halbringes 160 auf die Stützgabel 30 sicher auszuschließen.

Weitere, nicht dargestellte Halbringe können in ihrer Konvexität zwischen derjenigen des Halbringes 60 und des Halbringes 160 liegen, jedoch sind auch Halbringe mit noch größerer Konvexität als der Halbring 160 ohne weiteres mit der Stützgabel kuppelbar. Es versteht sich, daß alle Halbringe aus reinem Polyethylen natur hergestellt sind und einteilig gefertigt sind.

## Patentansprüche

1. Fixationsvorrichtung zur Verwendung an einem Extensions- und Repositionsgerät zum Einrichten einer Extremität, bestehend aus einer Stützgabel (30) aus einem sterilisierbaren, röntgendurchlässigen Kunststoff für die Extremität und einem Verbindungselement (60), welches an seinen beiden freien Enden je ein Kupplungsteil (67a, 69a) aufweist, und wobei die Stützgabel aus einem unteren Stiel (32), an welchem Halteteile zum Festlegen der Stützgabel an einem Support (600) des Gerätes befestigt sind, sowie einem seitlich vorstehenden Stützarm (36) und einem sich im wesentlichen parallel zum Stiel erhebenden Anlagearm (34) besteht, wobei an dem freien Ende des Anlagearmes und an dem freien Ende des Stützarmes je ein Gegenkupplungsteil (37, 39) ausgebildet sind, dadurch gekennzeichnet, daß das Verbindungselement (60) ein starrer, aus einem sterilisierbaren, röntgendurchlässigen Kunststoff bestehender Halbring ist, der den vom Stützarm (36) und dem Anlagearm (34) gebildeten Ringabschnitt zu einem geschlossenen Ring ergänzt, und daß die an den freien Enden des Halbringes ausgebildeten Kupplungsteile (67a, 69a) und die Gegenkupplungsteile (37, 39) als lösbare Steckverbindung ausgebildet sind.

2. Vorrichtung nach Anspruch 1, dadurch gekennzeichnet, daß der Halbring derartig nach außen konvex gebogen ist, daß die Richtungen (66, 68) seiner freien Enden einen spitzen Winkel einschließen.

3. Vorrichtung nach Anspruch 1, dadurch gekennzeichnet, daß der Halbring flach gebogen ist, derart, daß die Richtungen seiner freien Enden einen stumpfen Winkel einschließen (Fig. 8).

4. Vorrichtung nach einem der vorstehenden Ansprüche, dadurch gekennzeichnet, daß der Anlagearm (34) relativ zum Stiel (32) seitlich in Richtung des Stützarmes (36) versetzt an den Stiel angeformt ist.

5. Vorrichtung nach einem der vorstehenden Ansprüche, dadurch gekennzeichnet, daß der Kunststoff reines Polyäthylen natur ist.

6. Vorrichtung nach einem der vorstehenden Ansprüche, dadurch gekennzeichnet, daß der Stützarm (36) eine breite, abgerundete Auflagefläche für die Extremität aufweist.

7. Vorrichtung nach einem der vorstehenden Ansprüche, dadurch gekennzeichnet, daß die Querschnittsfläche des Stützarmes im wesentlichen ein liegendes Rechteck mit stark abgerundeten Ecken ist.

8. Vorrichtung nach einem der vorstehenden Ansprüche, dadurch gekennzeichnet, daß in den ·Stützarm mehrere Metallstücke (80, 81, 82) seitlich nebeneinander eingelassen sind.

9. Vorrichtung nach einem der vorstehenden Ansprüche, dadurch gekennzeichnet, daß in den Anlagearm (34) mehrere Metallstücke (86, 87) übereinander eingelassen sind.

10. Vorrichtung nach Anspruch 8 und 9, dadurch gekennzeichnet, daß in den Stützarm und in den Anlagearm eine verschiedene Anzahl von Metallstücken eingelassen sind.

11. Vorrichtung nach einem der Ansprüche 8 bis 10, dadurch gekennzeichnet, daß die Metallstücke aus Kupfer bestehen.

12. Vorrichtung nach einem der Ansprüche 8 bis 11, dadurch gekennzeichnet, daß die Metallstücke in dem Stützarm eine andere Größe haben als die Metallstücke im Anlagearm.

13. Vorrichtung nach einem der vorstehenden Ansprüche, dadurch gekennzeichnet, daß die Kupplungsteile an den Halbring angeformte Osen mit Augen sind, deren Innendurchmesser im wesentlichen gleich dem Außendurchmesser je eines Zapfens ist, der aus jedem Gegenkupplungsteil vorsteht.

14. Vorrichtung nach Anspruch 13, dadurch gekennzeichnet, daß jedes Gegenkupplungsteil aus einer an den Stützarm und an den Anlagearm angeformten Öse mit einem Auge besteht, wobei der separate Zapfen (40) durch das Auge der Kupplungsteile und der Gegenkupplungsteile steckbar ist.

15. Vorrichtung nach einem der vorstehenden Ansprüche, dadurch gekennzeichnet, daß der Stützarm, der Anlagearm und der Halbring neben den Kupplungs- und Gegenkupplungsteilen radial einwärts abgeflacht sind.

## Claims

1. A locator device for employment on an apparatus for extension aand repositioning for the setting of an extremity, consisting of a supporting fork (30) of a sterilizable plastics permeable to X-rays for the extremity and a connecting element (60) which exhibits at each of its free ends a coupling part (67a, 69a), where the supporting fork consists of a lower post (32) to which are fastened retainer parts for fixing the supporting fork onto a bracket (600) on the apparatus as well as a supporting arm (36) projecting sideways and a buffer arm (34) rising essentially in parallel with the post, and where at the free end of the buffer arm and at the free and of the supporting arm there is made in each case a countercoupling part (37, 39), characterized in that the connecting element (60) is a rigid halfring consisting of a sterilizable plastics permeable to X-rays, which completes the portion of ring formed by the supporting arm (36) and the buffer arm (34) into a closed ring, and that the coupling parts (67a, 69a) formed at the free ends of the half-ring and the countercoupling parts (37, 39) are made as releasable plug connections.

2. A device as in Claim 1, characterized in that the half-ring is bent outwards converty in such a way that the directions (66, 68) of its free ends include an acute angle.

3. A device as in Claim 1, characterized in that the half-ring is bent flat in such a way that the directions of its free ends include an obtuse angle (Figure 8).

4. A device as in one of the preceding Claims, characterized in that the buffer arm (34) relatively to the post (32) is shaped offset from the post sideways in the direction of the supporting arm (36).

5. A device as in one of the preceding Claims, characterized in that the plastics is of the nature of pure polyethylene.

6. A device as in one of the preceding Claims, characterized in that the supporting arm (36) exhibits a wide rounded bearing surface for the extremity.

7. A device as in one of the preceding Claims, characterized in that the cross-sectional area of the supporting arm is essentiaily a horizontal rectangle having heavily rounded corners.

8. A device as in one of the preceding Claims, characterized in that a number of pieces of metal (80, 81, 82) side by side are let into the supporting arm.

9. A device as in one of the preceding Claims, characterized in that a number of pieces of metal (86, 87) one above the other are let into the buffer arm (34).

10. A device as in Claim 8 and 9, characterized in that a different number of metal pieces are let into the supporting arm and into the buffer arm.

11. A device as in one of the Claims 8 to 10, characterized in that the metal pieces consist of copper.

12. A device as in one of the Claims 8 to 11, characterized in that the metal pieces in the supporting arm having a different size from the metal pieces in the buffer arm.

13. A device as in one of the preceding Claims, characterized in that the coupling parts are ears moulded onto the half-ring having eyes the inner diameter of which is essentially equal to the outer diameter of each pin which projects from each countercoupling part.

14. A device as in Claim 13, characterized in that each countercoupling part consists of an ear with an eye moulded onto the supporting arm and onto the buffer arm, so that the separate pin (40) can be plugged through the eyes in the coupling parts and in the countercoupling parts.

15. A device as in one of the preceding Claims, characterized in that the supporting arm, the buffer arm and the half-ring next to the coupling and countercoupling parts are flattened radially inwards.

## Revendications

1. Dispositif de fixation pour utilisation sur un appareil d'extension et de repositionnement en vue de la reposition d'une extrémité, constitué d'une fourche d'appui (30) en matière plastique stérilisable perméable aux rayons X, pour l'extrémité, et d'un élément de liaison (60) qui présente sur ses deux extrémités libres un demi-accouplement (67a, 69a), la fourche d'appui étant constituée d'une tige inférieure (32), sur laquelle sont fixées des pièces de retenue permettant la fixation de cette fourche d'appui sur un support (600) de l'appareil, ainsi que d'un bras d'appui (36) dépassant latéralement et d'un bras de repos (34) qui se soulève pratiquement de manière parallèle à la tige, un demi-accouplement opposé (37, 33) étant formé à l'extrémité libre du bras de repos et un à l'extrémité libre du bras d'appui, caractérisé en ce que l'élément de liaison (60) est un demi-anneau rigide, constitué d'une matière plastique stérilisable perméable aux rayons X, qui complète la partie d'anneau formée par le bras d'appui (36) et le bras de repos (34) de manière à constituer un anneau fermé, et en ce que les demi-accouplements (67a, 69a) formés sur les extrémités libres du demi-anneau et les demi-accouplements opposés (37, 39) sont conçus en tant que raccords à em-boîtement amovibles.

2. Dispositif conforme à la revendication 1, caractérisé en ce que le demi-anneau est courbé de manière convexe vers l'extérieur de telle manière que les directions (66, 68) de ses extrémités libres forment un angle aigü

3. Dispositif conforme à la revendication 1, caractérisé en ce que demi-anneau en arc surbaissé, tel que les directions de ses extrémités libres forment un angle obtus (Fig. 8).

4. Dispositif conforme à l'une quelconque des revendications précédentes, caractérisé en ce que, le bras de repos (34) est formé sur la tige (32), latéralement par rapport à celle-ci, en étant décalé en direction du bras d'appui (36).

5. Dispositif conforme à l'une quelconque des revendications précédentes, caractérisé en ce que la matière plastique est un polyéthylène pur nature.

6. Dispositif conforme à l'une quelconque des revendications précédentes, caractérisé en ce que le bras d'appui (36) présente une large surface d'appui à angles arrondis pour l'extrémité.

7. Dispositif conforme à l'une quelconque des revendications précédentes, caractérisé en ce que la section du bras d'appui forme pratiquement un rectangle horizontal à angles fortement arrondis.

8. Dispositif conforme à l'une quelconque des revendications précédentes, caractérisé en ce que plusieurs pièces métalliques (80, 81, 82) sont encastrées dans le bras d'appui où elles sont disposées latéralement près les unes des autres.

9. Dispositif conforme à l'une quelconque des revendications précédentes, caractérisé en ce que plusieurs pièces métalliques (86, 87) sont encastrées les unes au-dessus des autres dans le bras de repos (34).

10. Dispositif conforme aux revendications 8 et 9, caractérisé en ce qu'un nombre différent de pièces métalliques est encastré dans le bras d'appui et dans le bras de repos.

11. Dispositif conforme à l'une des revendications 8 à 10, caractérisé en ce que les pièces métalliques sont en cuivre.

12. Dispositif conforme à l'une des revendications 8 à 11, caractérisé en ce que les pièces métalliques encastrées dans le bras d'appui ont une dimension autre que celles encastrées dans le bras de repos.

13. Dispositif conforme à l'une quelconque des revendications précédentes, caractérisé en ce que les demi-accouplements sont des anneaux avec oeillets formés sur le demi-anneau, dont le diamètre interne est pratiquement égal au diamètre extérieur d'un tenon qui dépasse de chaque demi-accouplement opposé.

14. Dispositif conforme à la revendication 13, caractérisé en ce que chaque demi-accouplement opposé peut aussi être constitué d'un anneau avec oeillet, formé sur le bras d'appui et sur le bras de repos, le tenon séparé (40) pouvant être enfiché à travers l'oeillet des demi-accouplements et des demi-accouplements opposés.

15. Dispositif conforme à l'une quelconque des revendications précédentes, caractérisé en ce que le bras d'appui, le bras de repos et le demi-anneau sont nivelés radialement vers l'intérieur à côté des demi-accouplements et des demi-accouplements opposés.

FIG. 1

FIG. 5

FIG. 6

FIG. 3

FIG. 2

FIG. 4

0 080 193

FIG. 7

FIG. 8